# EUROPEAN PATENT APPLICATION

(11) **EP 2 145 611 A1**
(43) Date of publication of application: **20.01.2010**
(21) Application number: 08740836.5
(22) Date of filing: 24.04.2008
(51) Int. Cl.: A61F 5/451, A61F 5/44

(54) **ABSORPTIVE ARTICLE, ABSORPTIVE ARTICLE SYSTEM, AND CARE SYSTEM**

(30) Priority: 26.04.2007 JP 2007116569
(71) Applicant: Uni-charm Corporation, Ehime 799-0111 (JP)
(72) Inventor: WADA, Ichiro, Kanonji-shi Kagawa 769-1602 (JP); SUZUKI, Miou, Kanonji-shi Kagawa 769-1602 (JP)
(74) Representative: Eke, Philippa Dianne
(86) International application number: PCT/JP2008/057974
(87) International publication number: WO 2008/136363

(57) **Abstract**

An absorptive article which includes a locking part disposed on one end in the longitudinal direction so as to face the side of a wearing object and a locking part disposed on the other end in the longitudinal direction so as to face a side opposite to the wearing object. The care system includes the first locking part (251) disposed at an input terminal (250) fitted to an output terminal part (115) disposed on one end in the longitudinal direction (X) of the urine pad (100) so as to face the side of the wearing person and the second locking part disposed on the surface of a side opposite to the side of the wearing person, that is the other end in the longitudinal direction (X) of the urine pad (100). The urine pad (100) is used while the first locking part (251) is locked to a beltlike member put on the waist of the wearing person, and the second locking part is locked to the inner surface of an outer member so disposed as to cover the urine pad (100).

## Description

### TECHNICAL FIELD

The present invention relates to a care system including an absorbent article, an absorbent article system including the absorbent article, and an absorbent article used in these systems.

### BACKGROUND ART

Conventionally, an absorbent article such as a diaper has been known which disposes excretory substances such as menstrual blood, urine, feces excreted from those who require care such as elderly people who keep to their bed and cannot stand on their own legs, and the like. The absorbent article such as a diaper holds excretory substances such as urine excreted so that the excretory substances are not leaked outside of the absorbent article.

Here, in a case where smell, germ, and the like are occurred from excretory substances held in the absorbent article, it exerts a harmful influence to those who require care. Furthermore, a caretaker needs to replace the absorbent article holding the excretory substances, which situation increases the burden on care.

As for the foregoing, a care system has been proposed which is composed of a urine receiver, an aspirating tube connecting to the urine receiver, and aspirating configuration placed outside of the urine receiver (for example, Japanese Unexamined Patent Application, First Publication No. 2007-44494). According to the care system disclosed in Japanese Unexamined Patent Application, First Publication No. 2007-44494, the urine receiver receives excretory substances such as urine excreted from those who require care, and then the urine thus received by the urine receiver is aspirated outside of the urine receiver by the aspirating configuration. Based on the abovementioned configuration, excretory substances from those who require care are not leaked outside, and the excretory substances are not held in the urine receiver, whereby the urine receiver is kept clean. In addition, the urine receiver can be applied to those who require care for a long period of time without replacing the urine receiver.

Here, the urine receiver described in Japanese Unexamined Patent Application, First Publication No. 2007-44494 is disposed on the inside of the T-belt which is composed of a torso belt disposed circularly along a beltline of the wearer and a groin belt coupled with the torso belt. More specifically, the urine receiver is attached on the inside of the groin belt of the T-belt. Therefore, there may be the case in which the urine receiver cannot be applied so as to be fitted to a body according to the size of a body of the wearer.

In addition, regarding a conventional sanitary napkin and a urine receiver, there is one which has an adhesive portion continuously from one end to the other end of a face of the sanitary napkin and the urine receiver, which faces an underwear, as a fixed portion to the underwear and the like. In a case where the sanitary napkin, the urine receiver, and the like having such an adhesive portion is fixed to the underwear and the like to use, there may be the case in which the sanitary napkin and the urine receiver are spaced apart from a body so that excretory substances are leaked when the underwear cannot follow body movements.

### DISCLOSURE OF THE INVENTION

### Problems to be Solved by the Invention

The present invention has an object to provide a care system configuring an absorbent article, during wear, having a locking portion disposed on one end in a longitudinal direction so as to face a side of a wearer and a locking portion disposed on the other end in a longitudinal direction facing an opposite side from the wearer.

In addition, the present invention has an object to provide an absorbent article system configuring an absorbent article, during wear, having a locking portion disposed on one end in a longitudinal direction so as to face a side of a wearer and a locking portion disposed on the other end in a longitudinal direction facing an opposite side from the wearer.

In addition, the present invention has an object to provide an absorbent article, during wear, having a locking portion disposed on one end in a longitudinal direction on a side of a wearer and a locking portion disposed on the other end in a longitudinal direction on an opposite side of the wearer.

### Means for Solving the Problems

In a first aspect of the invention, a care system is provided, including: an absorbent article formed to be an elongated shape and disposed so as to cover an excretion portion of a wearer; a tube connected to the absorbent article, from which liquid excreted from the excretion portion can be discharged; a container connected to the tube, in which the liquid discharged from the absorbent article can be collected; a pump directly or indirectly coupled with the tube to thereby deliver the liquid to the container; a sensor disposed on the absorbent article to detect that the liquid is excreted onto the absorbent article; an output terminal to output detection result information from the sensor; an input terminal to connect with the output terminal and input the detection result information; and a control means for controlling the pump based on the detection result information inputted to the input terminal, in which the output terminal is disposed on one end in a longitudinal direction of the absorbent article, a first locking portion facing the wearer is disposed on the input terminal with the input terminal being connected to the output terminal, and a second locking portion is disposed on an opposite side from the wearer of the absorbent article on the other end of the absorbent article.

In a second aspect of the present invention, the care system according to the fist aspect is provided, further including: a band-shaped member disposed circularly, so as to be along an outer circumference of the wearer; and an outer member disposed so as to cover the absorbent article, in which the first locking portion is locked with the band-shaped member, and the second locking portion is locked with the outer member.

In a third aspect of the present invention, the absorbent article used in the care system according to first or second aspect is provided, including: a connecting portion connected to a tube, from which liquid excreted from the excretion portion can be discharged; a sensor to detect the liquid excreted onto the absorbent article; an output terminal to output detection result information from the sensor, which is disposed on one end of the absorbent article in a longitudinal direction; and a second locking portion disposed on an opposite side from the wearer of the absorbent article on the other end of the absorbent article.

In a fourth aspect of the present invention, the absorbent article according to the third aspect is provided, in which an input terminal in which the first locking portion is disposed can be connected with the output terminal, and the first locking portion is disposed so as to face the wearer with the input terminal being connected to the output terminal.

In a fifth aspect of the present invention, the absorbent article system is provided, including: a band-shaped member disposed circularly so as to be along an outer circumference of a wearer having an excretion portion; an elongated absorbent article disposed to cover the excretion portion of the wearer; an outer member disposed so as to cover the absorbent article; and a locking member coupled with one end of the absorbent article in a longitudinal direction, and having a first lock locked with the band-shaped member, in which a second locking portion locked with the outer member is disposed on a face disposed on the outer member of the other end of the absorbent article in a longitudinal direction.

In a sixth aspect of the present invention, the absorbent article system according to the fifth aspect in provided, in which the locking member is coupled in such a manner that the first locking portion faces the band-shaped member on one end of the absorbent article in the longitudinal direction, the first locking portion is locked with the band-shaped member, the second locking portion on the absorbent article is locked with the outer member, the absorbent member locked with the outer member by the second locking portion is disposed so as to cover the excretion portion of the wearer, and the outer member is disposed so as to cover the absorbent article locked with the outer member by the second locking member.

In a seventh aspect of the present invention, the absorbent member used in the absorbent article according to the fifth or sixth aspect is provided, including: a coupling portion which is disposed on one end of the absorbent article in a longitudinal direction, with which the locking member is coupled so that the first locking portion is disposed on the side of the wearer; and a second locking portion disposed on the outer member of the other end of the absorbent article in the longitudinal direction.

In an eighth aspect of the present invention, an absorbent article which is formed to be an elongated shape and disposed so as to cover an excretion portion of a wearer is provided, including: a first face disposed on the wearer when worn; and a second face disposed on an opposite side of the wearer, in which a first locking portion is disposed on an end of the absorbent article in a longitudinal direction on the first face, and a second locking portion is disposed on the other end of the absorbent article in the longitudinal direction on the second face.

In a ninth aspect of the present invention, the absorbent article according to the eighth aspect is provided, in which on the condition that the absorbent article is disposed to cover the excretion portion of the wearer, and an outer member is disposed so as to cover the absorbent article, the first locking portion is directly or indirectly locked with the wearer, and the second locking portion is locked with the outer member.

Here, an absorbent article of the present invention includes an article which can absorb liquid mechanically as well as an article having an absorber with liquid retention capability.

In addition, being locked indirectly to a wearer also refers to being locked by a locked member disposed so as to fit to a wearer.

### Effects of the Invention

The present invention can provide a care system configuring an absorbent article, during wear, having a locking portion disposed on one end in a longitudinal direction so as to face a side of a wearer and a locking portion disposed on the other end in a longitudinal direction facing an opposite side from the wearer.

In addition, the present invention can provide an absorbent article system configuring an absorbent article, during wear, having a locking portion disposed on one end in a longitudinal direction so as to face a side of a wearer and a locking portion disposed on the other end in a longitudinal direction facing an opposite side from the wearer.

In addition, the present invention can provide an absorbent article, during wear, having a locking portion disposed on one end in a longitudinal direction on a side of a wearer and a locking portion disposed on the other end in a longitudinal direction on an opposite side of the wearer.

### BRIEF DESCRIPTION OF THE DRAWINGS

FIG. 1 is a schematic diagram showing a care system according to the present invention;
FIG. 2A is a plain view showing a configuration of the urine pad in FIG. 1;
FIG. 2B is a plain view showing a sensor member in FIG. 2A and an input terminal attached with the sensor member;
FIG. 3 is a back side view showing a configuration of the urine pad in FIG. 1;
FIG. 4 is a diagram showing a wearing procedure when a person is wearing the urine pad in FIG. 1;
FIG. 5 is a diagram showing a wearing procedure when a person is wearing the urine pad in FIG. 1;
FIG. 6 is a diagram showing a wearing procedure when a person is wearing the urine pad in FIG. 1;
FIG. 7 is a diagram showing a wearing condition when a person is wearing the urine pad in FIG. 1;
FIG. 8 is a plain view showing a configuration of the urine pad according to the second embodiment; and
FIG. 9 is a back side view showing a configuration of the urine pad according to the second embodiment.

### PREFERRED MODE FOR CARRYING OUT THE INVENTION

### 1. First Embodiment

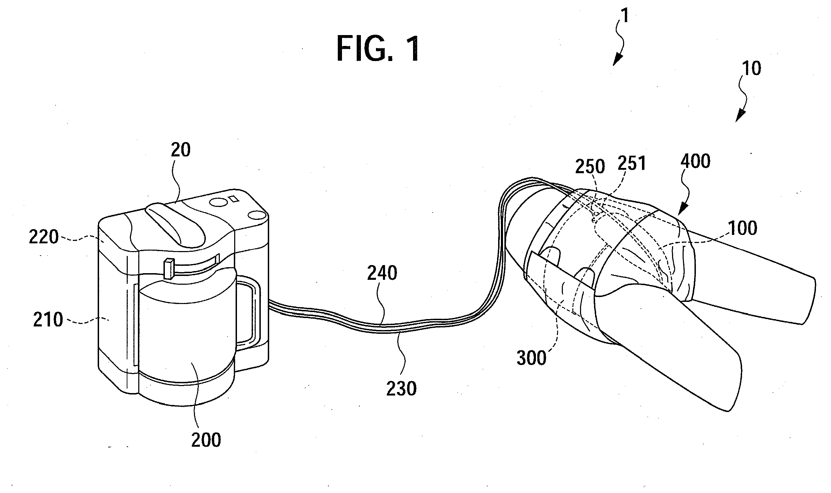

A care system 1 of an embodiment of the present invention is described with reference to FIGS. 1 to 7.

### 1.1 Care System

As showing in FIG. 1, a care system 1 has an absorbent article system 10 including a urine pad 100 and a device 20 including a container 200 and a pump 210.

### 1.2 Device

The device 20 includes the container 200, the pump 210, CPU 220, a tube 230, and a wire 240.

The container 200 is a resin container holding liquid such as urine excreted to the urine pad 100. More specifically, the container holds liquid such as urine excreted to the urine pad 100 via the tube 230.

The pump 210 delivers to the container 200 liquid such as urine excreted to the urine pad 100 via the tube 230. The pump 210 is indirectly connected with the container 200 via the tube 230. The pump 210 is driven based on an instruction from the CPU 220. More specifically, the pump 210 aspirates are in the container 200 based on the instruction from the CPU 220. Thus, liquid such as urine excreted to the urine pad 100 is aspirated in the container 200 via the tube 230. The liquid such as urine excreted to the urine pad 100 is delivered to the container 200 via the tube 230 by the pump 210 thus driven.

The CPU 220 as a control means controls the pump 210 based on a prescribed signal as a detection result information from a urine sensor 112 which is described later. More specifically, the CPU 220 drives the pump 210 by receiving a detection signal outputted from an output terminal portion 115, signal which indicates that the urine sensor 112 detects that urine is excreted.

The tube 230 is a tube-shaped member which can be transformed flexibly. The tube 230 is disposed so as to connect the urine pad 100 with the container 200. More specifically, the tube 230 is connected in such a manner that liquid such as urine excreted to the urine pad 100 can be delivered to the container 200. More specifically, the tube 230 is connected with a tube connecting portion 121 on the urine pad 100 (described later) in such a manner that the tube 230 is inserted to an opening of the container 200.

The wire 240 electrically connects a sensor member 100 with the CPU 220. More specifically, it is connected with the CPU 220 through an input terminal 250 (described later) and the wire 240. The input terminal 250 and the wire 240 are connected with the output terminal portion 115 which is formed as an output terminal on an end of the sensor member 110. The wire 240 serves as a path transmitting to the CPU 220 a signal such as a detection signal outputted from the output terminal portion 115 of the sensor member 110.

The input terminal 250 is disposed on one end of the wire 240 and electrically connected with the output terminal portion 115 which is described later. More specifically, the input terminal 250 is installed so as to tuck the output terminal portion 115. A connector (not shown) formed on the input terminal 250 is contacted with each connector formed in the output terminal portion 115 thereby electrically connecting with each other.

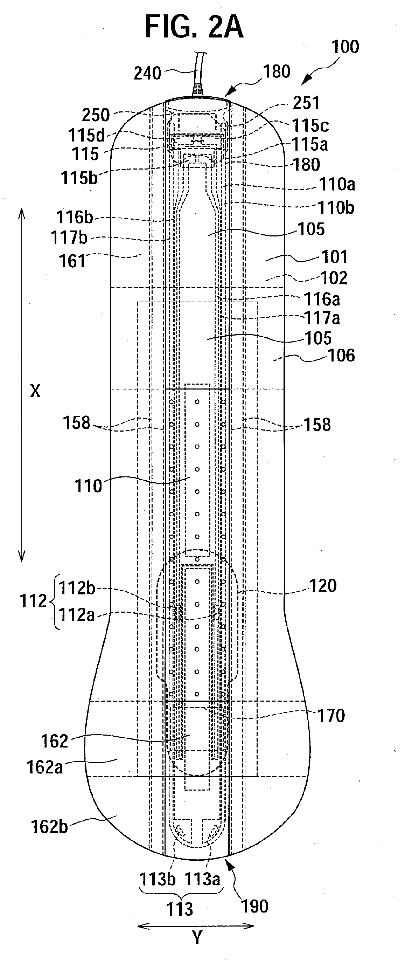

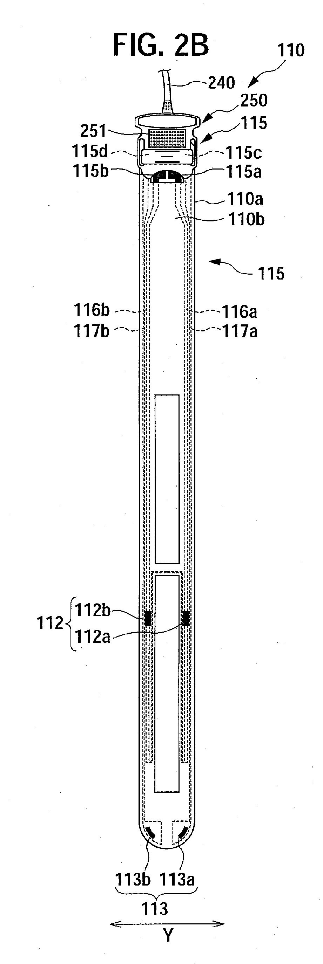

In addition, as shown in FIGS.2A and 2B, a first locking portin 251 is disposed on one face of the input terminal 250. The first locking portion 251 is disposed on one end of the urine pad 100 in a longitudinal direction with the input terminal 250 being attached with the output terminal portion 115. Then, the first locking portion 251 is disposed on the front side of a body and disposed so as to face the body, with the urine pad 100 being applied to the body. More specifically, the first locking portion 251 is locked at a portion of a band-shaped member 300 disposed along a beltline of the body, a portion which is disposed on the front side of the body. The urine pad 100 is locked with the band-shaped member 300 which is disposed in close contact with the body. That is, the input terminal 250 serves as an input portion for a detection signal and the like outputted from the output terminal portion 115, and serves as a locking member locking the urine pad 100 at the band-shaped member 300.

### 1.3 Absorbent Article System

An absorbent article system 10 includes the urine pad 100 as the absorbent article, the belt-shaped member 300, and an outer member 400. More specifically, the absorbent article system 100 includes a band-shaped member 300 disposed circularly along a beltline around a wearer's body to which the urine pad 100 is applied, the urine pad 100 in which one end in a longitudinal direction is locked at the band-shaped member 300 and is applied to the body so as to cover an excretion portion, and an outer member 400 disposed so as to cover the urine pad 100.

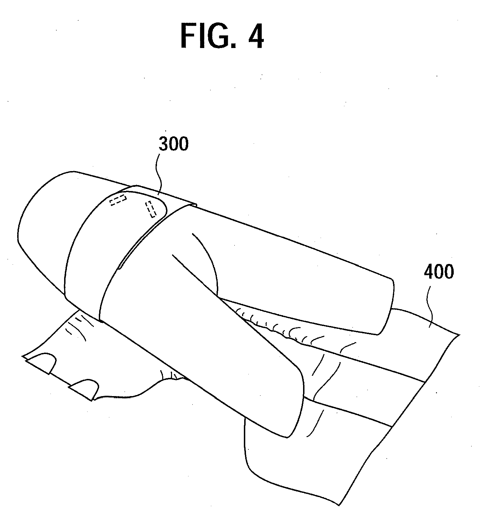

The band-shaped member 300 is the same in width and a member which can be elongated and contracted. In the present embodiment, the band-shaped member 300 is a sheet having stretching properties. A locking portion is disposed on one end of the belt-shaped member 300. As shown in FIGS 4 to 7, the band-shaped member 300 is disposed circularly along a beltline. In this condition, the locking portin is locked on the other end of the band-shaped member 300, and the band-shaped member 300 is applied so as to be along the beltline with the band-shaped member 300 being kept its circularity. The band-shaped member 300 may be configured to include a fabric. In this case, a hook member is preferably used for a first locking portion 251 (described later). The belt-shaped member 10 may be configured with a non-stretchable sheet.

The urine pad 100 is a vertically long absorbent article having a first face 101 disposed along a body and a second face 102 disposed opposite the body when a person is wearing the urine pad 100. Here, an absorbent article of the present invention includes an article which absorbs liquid mechanically as well as an article having an absorber with liquid retention capability. More specifically, a urine pad 100 which liquid is taken out to the outside by the device 20 of the present embodiment is included in an absorbent article.

In addition, the urine pad 100 includes a top sheet portion 105 with liquid permeability disposed on the first face 101, a back sheet portion 106 with liquid impermeability disposed on the second face 102, the sensor member 110 detecting urine and feces, a collecting portion 120 for collecting urines excreted, and a second locking portion 130 disposed on the second face 102.

The top sheet portion 105 is composed of a liquid permeability sheet disposed on the first face 101 of the urine pad 100. The top sheet portion 105 is disposed at the center of the urine pad 100 in a width direction Y so as to be extended in a longitudinal direction X. The top sheet portion 105 is disposed at a place contacted with the excretion portion with the urine pad 100 being applied to the body, and allows liquid such as urine excreted from an excretion portion of a wearer to be permeated. The top sheet portion 105 is not disposed in a vicinity of both ends of the urine pad 100 in a longitudinal direction X. The first sheet 161 and the second sheet 162 (described later) are disposed respectively on both ends of the urine pad 100 in a longitudinal direction X.

Gathers 158 are formed on both sides of the top sheet portion 105 in a width direction Y. Gathers 158 are deformed to be raised toward a body so that the gathers 158 are disposed so as to be directly contacted with groin in the state that the urine pad 100 is attached to the body. Thus, it can prevent from leaking liquid such as urine excreted from the excretion portion.

As described above, the first sheet 161 is disposed on the front edge 180 of the urine pad 100, and the second sheet 162 is disposed on the back edge 190.

The first sheet 161 is composed of a liquid impermeability sheet and disposed so as to cover the output terminal portion 115 of the sensor member 110. On the side of the front edge 180 of the urine pad 100, the first sheet 161 is not joined with the back sheet portion 106. Since the top sheet portion 105 and the back sheet portion 106 are spaced apart from each other, the output terminal portion 115 formed on an end of the sensor member 110 is exposed.

The second sheet 162 has a liquid permeability portion 162a and a liquid impermeability portion 162b. The second sheet 162 is joined with the back sheet portion 106 and the sensor member 110 in a vicinity of an end on the back edge 190 of the liquid impermeability portion 162a. Then, a cushion member 170 is disposed on a lower face of the liquid impermeability portion which is not joined with. The cushion member 170 serves as a cushion when the urine pad is in use, and also serves as a member which spaces the front edge 180 of the liquid impermeability portion 162a in a longitudinal direction X from the top sheet portion 105. Thus, the front edge 180 is spaced from the top sheet portion 105 by the cushion member 170, and the liquid impermeability portion 162a with which the back edge 190 is contacted blocks liquid such as urine flowing to the back edge 190.

The liquid permeability portion 162b is composed of a liquid impermeability sheet and disposed on the back edge 190 of the second sheet 162. The liquid permeability portion 162b is an area into which liquid such as urine does not flow and disposed an area in which a feces is excreted. The feces sensor 113 (described later) of the sensor member 110 is disposed on the lower face of the liquid permeability portion 162b.

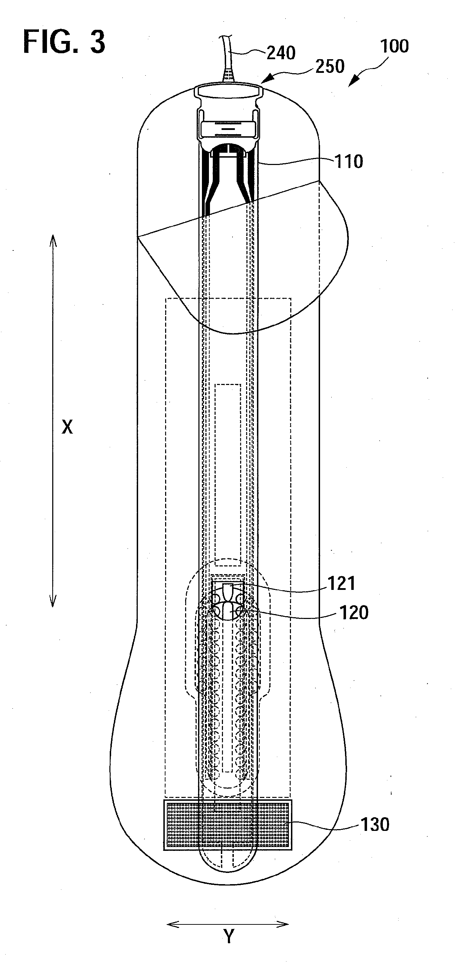

As shown in FIG. 3, the back sheet portion 106 is composed of a liquid impermeability sheet and disposed so as to configure the entire second face 102. The back sheet portion 106 prevents liquid such as urine excreted from an excretion portion of a wearer from being permeated, and delivers liquid such as urine to the collecting portion 120 (described later). The second locking portion 130 is disposed on the back edge 190 of the back sheet portion 106.

As shown in FIGS. 2A and 2B, the sensor member 110 includes the output terminal portion 115, a urine sensor 112, and the feces sensor 113. The output terminal portion 115 is formed at an end of the front edge 180 of the sensor member 110. The urine sensor 112 is formed between a center portion of the sensor member 110 in a longitudinal direction X and the back edge 190. In addition, the urine sensor 112 is in a vicinity of the collecting portion 120 and disposed on the front edge 180. The feces sensor 113 is disposed at an end of the back edge 190 of the sensor member 110.

The sensor member 110 is formed in such a manner that electrically conductive coating is printed on a film 110a which is flexible such as a polyethylene film. More specifically, electrically conductive coating is applied on one face of the film 110a in order to form electrodes 116a, 116b, 117a, and 117b, and a film 110b is laminated so as to cover the electrodes 116a, 116b, 117a, and 117b, thereby forming the sensor member 110.

Each electrodes 116a and 116b have first connectors 115a and 115b which configure the output terminal portion 115 and first electrode portions 112a and 112b which configure the urine sensor 112. The first connectors 115a and 115b are formed on one end of the electrodes 116a and 116b in a longitudinal direction X, respectively. The first electrode portions 112a and 112b are formed on the other end of the electrodes 116a and 116b in a longitudinal direction X, respectively.

The electrodes 117a and 117b have second connectors 115c an 115d which configure the output terminal portion 115 and second electrode portions 113a and 113b which configure the feces sensor 113. The second connectors 115c and 115d are formed on one end of the electrodes 117a and 117b in a longitudinal direction X, respectively. The second electrode portions 113a and 113b are formed on the other end of the electrodes 117a and 117b in a longitudinal direction X, respectively.

As described above, the output terminal portion 115 is formed on the front edge 180 of the sensor member 110 in a longitudinal direction X. When the urine pad 100 is not in use, the output terminal portion 115 is covered with the first sheet 161 and the back sheet portion 106. On the other hand, when the urine pad 100 is in use, the output terminal portion 115 is configured to be exposed outside.

The output terminal portion 115 has first connectors 115a and 115b which are electrically connected with the urine sensor 112 and second connectors 115c and 115d which are electrically connected with the feces sensor 113. An opening is formed at the locations which correspond to the first connectors 115a and 115b and the second connectors 115c and 115d of the film 110b, respectively. That is, in the sensor member 110, the first connectors 115a and 115b, and the second connectors 115c and 115d are exposed outside.

The first connectors 115a and 115b, and the second connectors 115c and 115d thus exposed outside are contacted respectively with each connector (not shown) formed on the input terminal 250 to be electrically connected therewith. Thus, a detection signal and the like outputted from the first connectors 115a and 115b and the second connectors 115c and 115d are outputted to the CPU 220 via each connector disposed in the input terminal 250.

The urine sensor 112 includes the first electrode portions 112a and 112b. An opening is formed at the locations which correspond to the first electrode portions 112a and 112b, respectively. That is, in the sensor member 110, the first electrode portions 112a and 112b are exposed outside, respectively.

The urine sensor 112 detects urine in such a manner that the first electrode portions 112a and 112b configuring the urine sensor 112 are electrically connected with each other. More specifically, the urine sensor 112 detects urine in such a manner that the first electrode portions 112a and 112b are electrically connected with each other by urine excreted.

Since the urine sensor 112 is disposed in a vicinity of an entrance of the collecting portion 120 where liquid such as urine thus excreted is collected, the liquid such as urine thus excreted is collected at the collecting portion 120 through the urine sensor 112. Thus, the liquid such as urine thus excreted gets through the urine sensor 112, the urine sensor 112 detects urine in such a manner that the first electrode portions 112a and 112b configuring the urine sensor 112 are electrically connected with each other by urine excreted.

Since the first electrode portions 112a and 112b are electrically connected with each other by urine excreted, a detection signal as detection result information is outputted from the urine sensor 112. The detection signal is outputted from first connectors 115a and 115b and inputted to the CPU 220 via the input terminal 250 and the wire 240. Since the detection signal is inputted, the CPU actuates the pump 210.

The feces sensor 113 includes the second electrode portions 113a and 113b. An opening is formed at the locations which correspond to the second electrode portions 113a and 113b of the film 110b, respectively. That is, in the sensor member 110, the second electrode portions 113a and 113b are exposed outside, respectively.

The feces sensor 113 detects feces in such a manner that the second electrode portions 113a and 113b configuring the feces sensor 113 are electrically connected with each other. More specifically, the feces sensor 113 detects feces in such a manner that the second electrode portions 113a and 113b are electrically connected with each other by liquid included in feces.

The feces sensor 113 is disposed where urine does not come in and feces are excreted. In addition, the liquid permeability portion 162b of the second sheet 162 is disposed on an upper face of the feces sensor 113. That is, in a case where a feces is excreted on the liquid permeability portion 162b from an excretion, the second electrode portions 113a and 113b configuring the feces sensor 113 are electrically connected with each other by liquid included in a feces, thereby enabling to detect feces thus excreted.

Since the second electrode portions 113a and 113b are electrically connected with each other, a detection signal as detection result information is outputted from the feces sensor 113. The detection signal is outputted from the second connectors 115c and 115d and inputted to the CPU 220 via the input terminal 250 and the wire 240. In a case where a detection signal is inputted, the CPU 220 turns on a light (not shown) which indicates that a feces was excreted.

The collecting portion 120 is stored and disposed between from the center portion to the back edge 190 of the urine pad 100 in a longitudinal direction. The collecting portion 120 is formed to be a rectangular solid in which the first face 101 is open. The collecting portion 120 is also a flexible resin member in which a plurality of protruding portions protruding from a bottom face located on the second face 102 to the first face 101 are formed. Because of the protruding portions, the collecting portion 120 is prevented from being collapsed under the weight of a body. Thus, the collecting portion 120 preferably collects liquid. Urine flows into the collecting portion 120 from the opening of the first face 101.

On a bottom face of the collecting portion 120, a tube connecting portion 121 having a liquid passing path penetrating the bottom face is formed. The tube 230 is connected with the tube connecting portion 121 in such a manner that liquid can be passed therein. Then, the pump 210 indirectly connected with the tube 230 is actuated, and urine collected by the collecting portion 120 is aspirated in the container 200.

The second locking portion 130 is disposed on the second face 102 of the urine pad 100. More specifically, the second locking portion 130 is disposed on the back edge 190 of the second face 102. More specifically, the second locking portion 130 is configured with a rectangular hook member which is long in a width direction Y, and is disposed in a vicinity of the back edge 190. The second locking member 130 is locked at an inner face of an outer member 400.

The outer member 400 is a diaper-shaped member. When a person wears the urine pad 100, the outer member 400 is disposed so as to cover the external side of the urine pad 100. On the inner face of the outer member 400, the second locking portion 130 is locked which is disposed on the back edge 190 of the second face 102 of the urine pad 100. The outer member 400 serves as a member which prevents an excretory substance leaked from the urine pad 100 from being leaked outside, and also serves as a locked portion of the urine pad 100.

### 1.4 Wearing procedure and Wearing condition

Based on FIGS. 4 to 7, a wearing procedure and a wearing condition of the urine pad 100 (an absorbent article) are described. Firstly, as shown in FIG. 4, the band-shaped member 300 is applied along a beltline of a body. By a locking member (not shown) disposed on one end of the band-shaped member 300, the band-shaped member 300 is applied circularly. Then, a wearer wearing the band-shaped member 300 is laid down on his/her back on the outer member 400 which is unfolded on the floor.

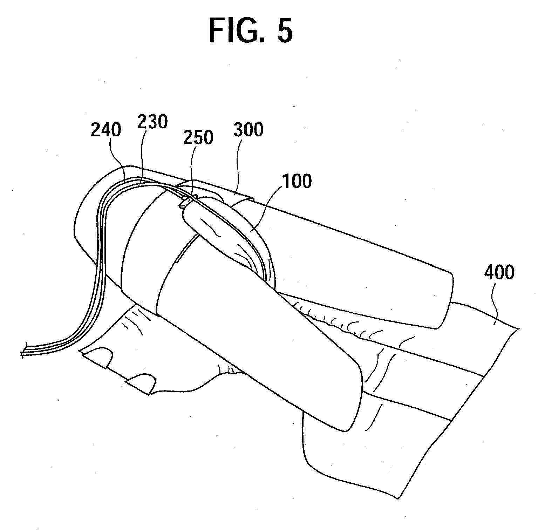

Then, as shown in FIG. 5, the urine pad 100, with the input terminal 250 being attached with the output terminal portion 115, is applied so as to cover the excretion portion. More specifically, firstly, the first locking portion 251 which is disposed at the input terminal 250 attached to the output terminal portion 115 is locked on a surface of the band-shaped member 300. Then, based on the position of the first locking portion 251 locked with the band-shaped member 300, a wearing position of the urine pad 100 is adjusted. Then, the second locking portion 130 which is disposed on the back edge 190 of the second face 102 of the urine pad 100 is locked on the inner face of the outer member 400. Here, the second locking portion 130 is locked at a position which is close to an excretion portion of the back side of a wearer. Here, the tube 230 and the wire 240 are disposed so as to be extended from a front side of the body.

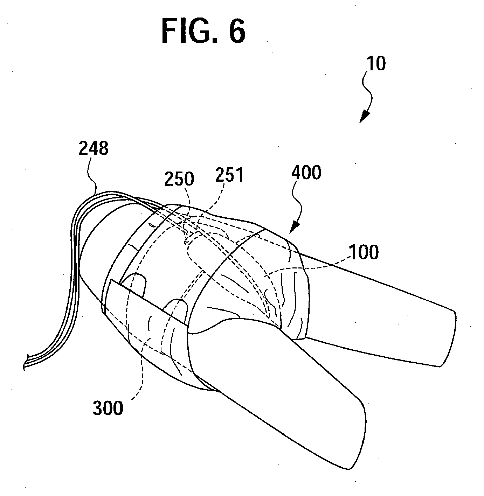

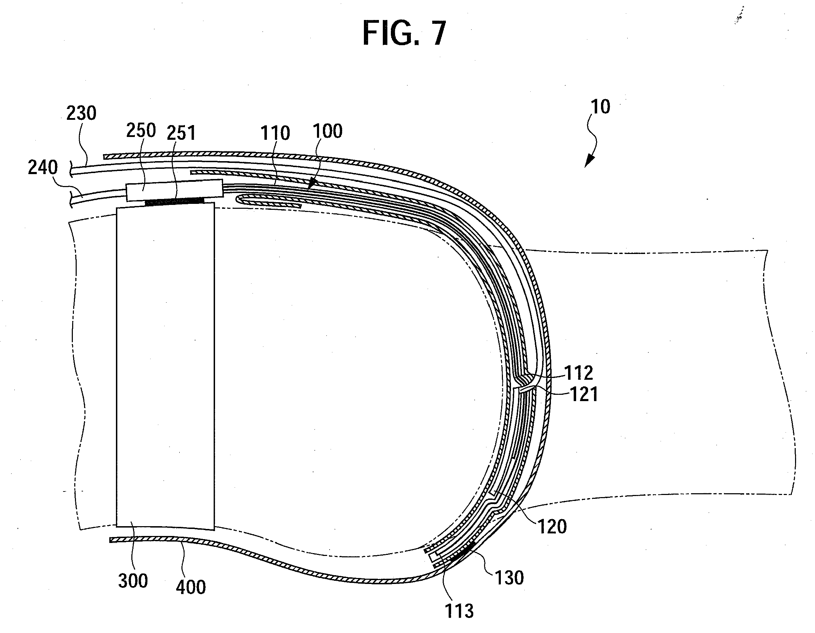

Then, as shown in FIGS. 6 and 7, the outer member 400 is attached to the body so as to cover the urine pad 100 disposed so as to cover the excretion portion. More specifically, the outer member 400 is locked with the band-shaped member 300 by the first locking portion 251, and is also attached to the body so as to cover the urine pad 100 which is locked at the inner face of the outer member 400 by the second locking portion 130. Thus, the urine pad 100 thus applied (an absorbent article system) follows body movements of the outer member 400 and the body (the body-shaped member 300). The outer member 400 thus applied prevents an excretory substance from being leaked, with the second locking portion 130 of the urine pad 100 being locked at the inner face thereof.

### 1.5 Effect

According to the present embodiment, the urine pad 100 can be easily applied to a wearer. According to the urine pad 100 of the present embodiment, the urine pad 100 can be applied to a wearer without moving a body of the wearer greatly. In addition, according to the urine pad 100 of the present embodiment, a position of the second locking portion can be easily readjusted.

In addition, according to the present embodiment, the urine pad 100 can be applied to a wearer in a preferred condition regardless of the body size of a wearer.

In addition, according to the present embodiment, the urine pad 100 can be applied so that the urine pad 100 can follow the body movements even though the wearer does not wear tight clothing.

In addition, according to the present embodiment, the urine pad 100 for care can be downsized. More specifically, a length of the urine pad can be shortened.

In addition, according to the present embodiment, the urine pad 100 can be locked by the second locking portion by wearing the outer member outside of the urine pad 100, and it can prevent from leaking out excretory substances which are leaked from the urine pad 100.

### 2. Second Embodiment

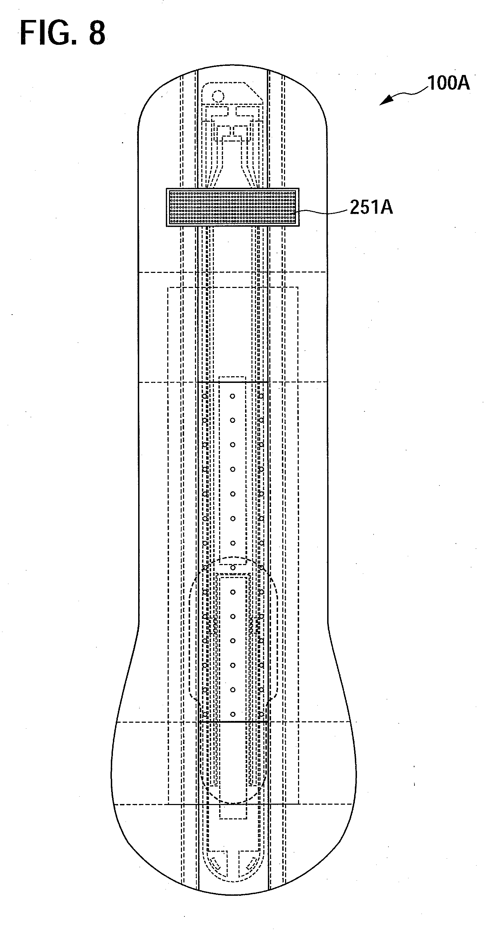

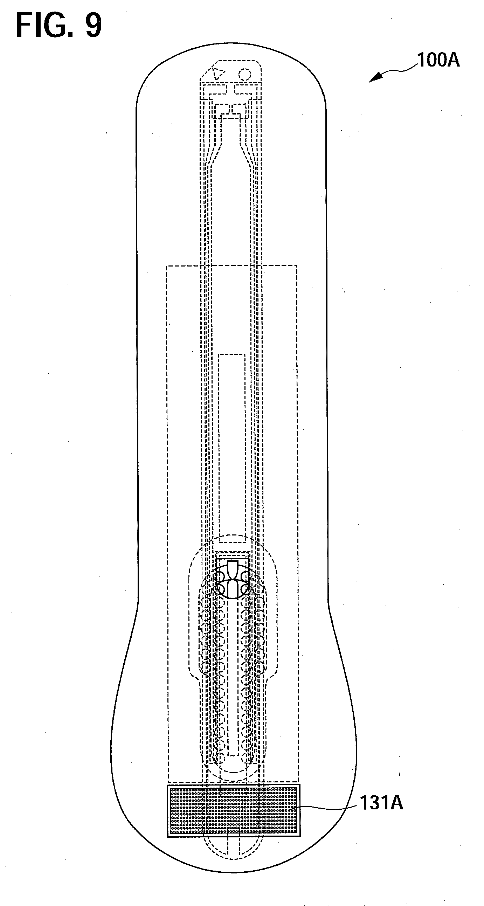

A urine pad 100A in the second embodiment is described with reference to FIGS. 8 and 9. As shown in FIGS. 8 and 9, the urine pad 100A in the present embodiment is different from the urine pad 100 (an absorbent article system) in the first embodiment in that the first locking portion is disposed on the urine pad 100A. In the following, as for the urine pad 100A, the differences from the urine pad 100 in the first embodiment are mainly described.

As shown in FIG. 8, the first locking portion 251A is disposed on the front edge 180 on the first face 101 of the urine pad 100A. The urine pad 100 in the first embodiment is locked with the abovementioned band-shaped member 300 by the first locking portion 251 which is disposed in the input terminal 250 attached to the output terminal portion 115. On the other hand, the urine pad 100A in the present embodiment is locked with the band-shaped member 300 and the like by a first locking portion 251A which is disposed on the urine pad 100A itself.

As shown in FIG. 9, a second locking portion 130A is disposed on the back edge 190 on the second face 102 of the urine pad 100A. That is, the urine pad 100A in the present embodiment has locking portions on both sides thereof in a longitudinal direction X. The locking portion on the front edge 180 is disposed on the first face 101, and the locking portion on the back edge 190 is disposed on the second face 102.

Here, the urine pad 100A in the present embodiment is applied to a wearer in the same procedure as the urine pad 100 in the first embodiment is. In addition, the urine pad 100A in the present embodiment makes the same effect with the urine pad 100 in the first embodiment, and also makes an effect that the urine pad 100A can be applied to a wearer without attaching a locking member having the first locking portion.

### 3. Others

The top sheet portion 105 is formed in the shape of a resin film having a plurality of liquid-passage pores, a net sheet having a plurality of mesh pores, nonwoven fabric having liquid permeability, fabric, or the like. The resin film or net sheet used may be any of those prepared from polypropylene (PP), polyethylene (PE), polyethylene terephthalate (PET), and so on. In addition, the nonwoven fabric used may be any of spunlaced nonwoven fabrics made of cellulose fibers such as rayon and synthetic resin fibers and air-through nonwoven fabrics made of the synthetic resin fibers. In addition, a material may be any of biodegradable natural products, such as polylactic acid, chitosan, and polyalginic acid. Furthermore, the top sheet portion 105 may be formed with a plurality of liquid-passage pores in the surface thereof, while a silicon- or fluorine-based water-repellent oil solution is applied thereon to provide a sheet having an external surface on which the body fluid can be hardly attached.

The back sheet portion 106 may be a material capable of preventing an excretory substance such as urine from being leaked out outside. In addition, the back sheet portion 106 may be made of a moisture-permeable raw material to make possible to decrease steamy conditions in wear, thereby improving the feeling of discomfort in wear. The materials for the back sheet portion 106 include a liquid-impermeable film mainly comprising polyethylene (PE), polypropylene (PP), or the like, an air-permeable film, and a composite sheet prepared by laminating a liquid-impermeable film on one side of a spun-bonded nonwoven fabric or the like. Preferably, a laminate of a hydrophilic nonwoven fabric or an impermeable plastic film with an impermeable plastic film can be used. Alternatively, it may be a SMS nonwoven fabric sandwiched by melt-blown nonwoven fabrics having high water-resisting property and high-strength spun-bonded nonwoven fabrics.

The collecting portion 120 can be manufactured using a flexible elastic material such as flexible polyethylene, silicon rubber, and the like. For example, the collecting portion 120 can be manufactured by molding the abovementioned material into a prescribed shape such as using injection molding.

In addition, in the abovementioned embodiment, although the first locking portion and the second locking portion are composed of a hook member, the present invention is not limited thereto, and a sheet with adhesive, a member which can be locked with a body and the like can be used as appropriate.

In addition, although, in the abovementioned embodiment, the urine pad as an absorbent article is described, the present invention is not limited thereto, and may be applied to a diaper, a sanitary napkin, and the like.

In addition, although, in the abovementioned embodiment, the outer member is a diaper-shaped member, the present invention is not limited thereto, and may be applied to cloth.

In addition, although, in the second embodiment, the urine pad used in the care system 1 as is the same as that in the first embodiment is described, the present invention is not limited thereto, and may be applied to a normal absorbent article.

## Claims

1. A care system comprising:
an absorbent article formed to be an elongated shape and disposed so as to cover an excretion portion of a wearer;
a tube connected to the absorbent article, from which liquid excreted from the excretion portion can be discharged;
a container connected to the tube, in which the liquid discharged from the absorbent article can be collected;
a pump directly or indirectly coupled with the tube to thereby deliver the liquid to the container;
a sensor disposed on the absorbent article to detect that the liquid is excreted onto the absorbent article;
an output terminal to output detection result information from the sensor;
an input terminal to connect with the output terminal and input the detection result information; and
a control means for controlling the pump based on the detection result information inputted to the input terminal,
wherein the output terminal is disposed on one end in a longitudinal direction of the absorbent article,
a first locking portion facing the wearer is disposed on the input terminal with the input terminal being connected to the output terminal, and
a second locking portion is disposed on an opposite side from the wearer of the absorbent article on the other end of the absorbent article.

2. The care system according to claim 1, further comprising:
a band-shaped member disposed circularly, so as to be along an outer circumference of the wearer; and
an outer member disposed so as to cover the absorbent article,
wherein the first locking portion is locked with the band-shaped member, and
the second locking portion is locked with the outer member.

3. The absorbent article used in the care system according to claim 1 or 2, comprising:
a connecting portion connected to a tube, from which liquid excreted from the excretion portion can be discharged;
a sensor to detect the liquid excreted onto the absorbent article; and
an output terminal to output detection result information from the sensor, which is disposed on one end of the absorbent article in a longitudinal direction,
wherein the second locking portion is disposed on an opposite side from the wearer of the absorbent article on the other end of the absorbent article.

4. The absorbent article according to claim 3,
Wherein an input terminal in which the first locking portion is disposed can be connected with the output terminal, and
the first locking portion is disposed so as to face the wearer with the input terminal being connected to the output terminal.

5. The absorbent article system, comprising:
a band-shaped member disposed circularly so as to be along an outer circumference of a wearer having an excretion portion;
an elongated absorbent article disposed to cover the excretion portion of the wearer;
an outer member disposed so as to cover the absorbent article; and
a locking member coupled with one end of the absorbent article in a longitudinal direction, and having a first lock locked with the band-shaped member,
wherein a second locking portion locked with the outer member is disposed on a face disposed on the outer member of the other end of the absorbent article in a longitudinal direction.

6. The absorbent article system according to claim 5,
wherein the locking member is coupled in such a manner that the first locking portion faces the band-shaped member on one end of the absorbent article in the longitudinal direction,
the first locking portion is locked with the band-shaped member,
the second locking portion on the absorbent article is locked with the outer member,
the absorbent member locked with the outer member by the second locking portion is disposed so as to cover the excretion portion of the wearer, and
the outer member is disposed so as to cover the absorbent article locked with the outer member by the second locking member.

7. The absorbent member used in the absorbent article according to claim 5, comprising:
a coupling portion which is disposed on one end of the absorbent article in a longitudinal direction, with which the locking member is coupled so that the first locking portion is disposed on the side of the wearer,
wherein a second locking portion is disposed on the outer member of the other end of the absorbent article in the longitudinal direction.

8. An absorbent article which is formed to be an elongated shape and disposed so as to cover an excretion portion of a wearer, comprising:
a first face disposed on the wearer when worn; and
a second face disposed on an opposite side of the wearer,
wherein a first locking portion is disposed on an end of the absorbent article in a longitudinal direction on the first face, and
a second locking portion is disposed on the other end of the absorbent article in the longitudinal direction on the second face.

9. The absorbent article according to claim 8,
Wherein on the condition that the absorbent article is disposed to cover the excretion portion of the wearer, and an outer member is disposed so as to cover the absorbent article, the first locking portion is directly or indirectly locked with the wearer, and
the second locking portion is locked with the outer member.
